# EUROPEAN PATENT SPECIFICATION

(11) **EP 2 164 500 B1**
(45) Date of publication and mention of the grant of the patent: **04.12.2013**
(21) Application number: 08753969.8
(22) Date of filing: 26.05.2008
(51) Int. Cl.: A61K 31/7068, A61K 31/495, A61K 31/513, A61K 31/706, A61P 17/06, A61P 25/14, A61P 29/00, A61P 9/10

(54) **THE USE OF ZEBURALINE FOR THE TREATMENT OF AUTOIMMUNE DISEASES OR IMMUNE REJECTION OF TRANSPLANTS**
VERWENDUNG VON ZEBURALIN ZUR BEHANDLUNG VON AUTOIMMUNKRANKHEITEN ODER IMMUNABSTOSSUNG VON TRANSPLANTATEN
UTILISATION DE ZÉBURALINE POUR LE TRAITEMENT DE MALADIES AUTOIMMUNITAIRES OU POUR UN REJET IMMUNITAIRE DE GREFFES

(30) Priority: 25.05.2007 SE 0701275; 07.06.2007 US 933478 P
(43) Date of publication of application: 24.03.2010
(73) Proprietor: Idogen AB, 223 61 Lund (SE)
(72) Inventor: SALFORD, Leif, 22362 Lund (SE); PERSSON, Bertil, 22475 Lund (SE); SJÖGREN, Hans, Olov, 22240 Lund (SE); WIDEGREN, Bengt, 22471 Lund (SE)
(74) Representative: Brann AB
(86) International application number: PCT/SE2008/000352
(87) International publication number: WO 2008/147283

(56) References cited:
- WO-A1-2006/116803
- WO-A2-03/012051
- WO-A2-03/072757
- WO-A2-2004/037159
- WO-A2-2004/041195
- WO-A2-2005/009349
- WO-A2-2007/016037
- WO-A2-2008/028193
- US-A1- 2007 042 976
- MELLOR A L ET AL: "IDO EXPRESSION BY DENDRITIC CELLS: TOLERANCE AND TRYPTOPHAN CATABOLISM", NATURE REVIEWS. IMMUNOLOGY, NATURE PUBLISHING GROUP, GB, vol. 4, no. 10, 1 October 2004 (2004-10-01), pages 762-774, XP009055502, ISSN: 1474-1733, DOI: 10.1038/NRI1457
- LIU ET AL: "Low dose Zebularine treatment enhances immunogenicity of tumor cells", CANCER LETTERS, NEW YORK, NY, US, vol. 257, no. 1, 27 September 2007 (2007-09-27), pages 107-115, XP022276694, ISSN: 0304-3835, DOI: 10.1016/J.CANLET.2007.07.013

## Description

### FIELD OF INVENTION

The invention relates to the use of zebularine(1-(β-D-Ribofuranosyl)-1,2-dihydropyrimidin-2-one), analogue or salts thereof for the manufacturing of a medicament to increase the amount of Indoleamine 2,3-dioxygenase (IDO) production in order to induce immunological tolerance in a method of treating a mammal in need thereof.

### BACKGROUND OF INVENTION

Organ transplantation is currently the treatment of choice for end-stage kidney, heart and liver diseases and is increasingly performed also in patients with failing other organs. However, despite advances in the often life-long immunosuppressive therapy and prophylaxis of infectious complications, rejection and severe infections still remain as problems after organ transplantation. In gene therapy, genes are in vivo transferred into viable cells of patients or in vitro whereafter the modified cells are transplanted to the patient resulting in continued production of the corresponding proteins as long as the producing cells survive and the transferred genes are not lost or silenced, e.g. by DNA methylation. As a rule additional proteins related to the vector used for gene transfer are also expressed. These proteins are foreign to the patient whose immune system mounts an immune response resulting in a high risk of killing of the expressing cells and loss of effect of the gene therapy.

It is well recognized that the optimal way of avoiding these problems of rejection of cells or organs expressing foreign antigens is to induce immunological tolerance to these antigens. In cases where an antigen selective non-responsiveness is physiologically developing, it is often dependent on a functioning IDO and administration of inhibitors of IDO breaks the state of unresponsiveness (Miki et al., 2001). Transfer of the IDO gene into allogeneic cells can lead to over expression of IDO and has been reported to result in a strong suppression of the rejection response to the allografts and permanent survival of the transferred cells without supporting immunosuppressive therapy.

Autoimmune diseases are widely spread and are causing a substantial proportion of the chronical illnesses in man. There is a great number of different autoimmune diseases, the most common being Rheumatoid arthritis, Diabetes type I, Psoriasis, Sjögrens syndrome, Multiple Sclerosis (MS), Crohns disease. There also exists a number of degenerative diseases that are likely to have autoimmune components and among these can be mentioned, arteriosclerosis, Parkinson's disease, ALS (Amyotrophic lateral sclerosis), dementia. In addition, there are situations when the immune system reacts very strongly in trauma like stroke and heart infarction and where the insult caused by lack of oxygen is enhanced by toxicity from the secondary immune reactivity. Another such situation where the immune system causes damage is after transplantation or gene therapy. The rejection of the transplant is mediated by the immune system and transplanted or genetically treated patients need rather strong immune suppressive drugs for the rest of there lives and these immune suppressive drugs cause several severe side effects. Zebularine is a cytidine analogue, and a DNA methyl transferase inhibitor that has been shown to be stable and not very toxic compared to azacytidine or 2'-deoxy-azacytidine (Cheng et al. 2003). Inhibitors of DNA methyl transferases have been used in preclinical and clinical trials as therapies against cancer. Genes inhibited by methylation that can favour tumour growth are tumour suppressor genes, apoptosis inducing genes, anti-angiogenetic genes, immune-stimulatory genes, and tumour antigens. In human bladder cancer cells, zebularine (100-500 µM) induces p16 gene expression (Cheng et al. 2004a) and when the bladder carcinoma cells grown in BALB/c mice were treated with zebularine (500-1000 mg/kg), tumour volume was reduced, and an in vivo p16 gene expression was induced (Cheng et al. 2004a). Zebularine can also change the expression of some other genes in cancer cells such as the tumour antigen MAGE-1 that is important to interact with the immune system (Cheng et al. 2004b, Liu et al. 2004.

Indoleamine 2,3-dioxygenase (IDO) degrades the indole moiety of tryptophan and initiates the production of neuroactive and immunoregulatory metabolites, collectively known as kynurenines. The functional expression of IDO by dendritic cells has emerged in recent years as a major mechanism of peripheral tolerance. IDO contributes to maternal tolerance in pregnancy, control of allograft rejection, and protection against autoimmunity, inflammatory pathology and allergy. IDO expression also serves a physiological mechanism by which malignancies induce immune tolerance (Uyttenhove et al. 2004; Mellor et al. 2004; Munn et al 2004;). The wide spectrum of physiopathological conditions in which IDO appears at work suggests that this suppressive system is frequently involved in physiological down regulation of T cell responses and resulting inflammatory responses.

### SUMMARY OF THE INVENTION

The invention relates to the use of zebularine(1-(β-D-Ribofuranosyl)-1,2-dihydropyrimidin-2-one), determined analogues thereof or salts thereof for the manufacturing of a medicament to increase the amount of Indoleamine 2,3-dioxygenase (IDO) production in order to induce immunological tolerance for treating diseases as specified in claim 1.

Indolamine dioxygenase(IDO) produces catabolites and metabolites from tryptophan, see below. The medicament of the invention induces immunological tolerance in a mammal and may be used to treat a number of disorders and diseases, such as those mentioned below.

Additionally, the invention relates to the use of at least zebularine, determined derivatives thereof or salts thereof for the manufacturing of a medicament for the treatment of an autoimmune disorder or disease or immune rejection of transplants as specified in claim 1 or gene therapeutically modified cells, wherein the treatment induces indolamine dioxygenase.

By providing such a medicament it is possible for the first time to induce immunological tolerance in an efficient way and thereby to enable the possibility to treat a number of diseases as defined below.

Zebularine or an analogue, or salts thereof may be used for the manufacturing of a medicament for the treatment of a disease selected from the group consisting of Achlorhydria, Acute hemorrhagic leukencephalitis, Addison's Disease, Alopecia Areata, Anemia, Pernicious Anti - Glomerular Basement Membrane Disease, Antiphospholipid Syndrome, Aplastic Anemia, Atopic Allergy, Autoimmune Atrophic Gastritis, Autoimmune Hearing Loss, Autoimmune hemolytic anemia, Autoimmune hypoparathyroidism, Autoimmune hypophysitis, Autoimmune Lymphoproliferative, Autoimmune Myocarditis, Autoimmune oophoritis, Autoimmune orchitis, Autoimmune Polyendocrinopathy-Candidiasis-Ectodermal-Dystrophy, Autoimmune Syndrome Type II, Polyglandular, Behcet Syndrome, Celiac Disease, Chagas Disease, Cholangitis, Sclerosing, Chronic Inflammatory Demyelinating Polyneuropathy, Chronic lymphocytic thyroiditis, Churg - Strauss Syndrome, Colitis, Ulcerative, Crohn's disease, Cryoglobulinemia, Cushing Syndrome, Dermatitis Herpetiformis, Dermatomyositis, Diabetes Mellitus (Insulin - Dependent), Diffuse Cerebral Sclerosis of Schilder, Encephalomyelitis, Autoimmune, Experimental (EAE), Epidermolysis Bullosa Acquisita, Erythematosis, Felty's Syndrome, Glomerulonephritis (IGA), Glomerulonephritis Membranous, Goodpasture Syndrome, Graves' Disease, Guillain - Barre Syndrome, Hamman-Rich syndrome, Hepatitis Autoimmune, Hepatitis Chronic Active, Idiopathic thrombocytopenia, Inflammatory Bowel Diseases, Insulin resistance - type B, Lambert - Eaton Myasthenic Syndrome, Lens-induced uveitis, Lichen Sclerosus et Atrophicus, Lupus Erythematosus Discoid, Lupus Erythematosus Systemic, Lupus Hepatitis, Lupus Nephritis, Lymphopenia, Meniere's Disease, Mixed Connective Tissue Disease, Mooren's ulcer, Mucocutaneous Lymph Node Syndrome, Multiple Sclerosis, Myasthenia Gravis, Myelitis Transverse, Myocarditis, Narcolepsy, Neuritis Autoimmune Experimental, Neuromyelitis Optica, Oculovestibuloauditory syndrome, Ophthalmia Sympathetic, Opsoclonus - Myoclonus Syndrome, Pancreatitis, Pemphigoid Bullous, Pemphigus foliaceous, Pemphigus Vulgaris, Polyarteritis Nodosa, Polychondritis Relapsing, Polyendocrinopathies Autoimmune, Polymyalgia Rheumatica, Polyradiculoneuropathy, Primary biliary cirrhosis, Purpura Thrombocytopenic Idiopathic, Raynauds, Reiter Disease, Rheumatic Fever, Rheumatoid Arthritis, Sarcoidosis, Scleroderma, Sjögren's Syndrome, Spondylitis Ankylosing, Stiff - Person Syndrome, Still's Disease Adult Onset, Takayasu's Arteritis, Temporal Arteritis, Thyrotoxicosis, Type B Insulin Resistance, Uveomeningoencephalitic Syndrome, Wegener's Granulomatosis, Vitiligo. In addition diseases that can partly be involved with autoimmune reactivity are arteriosclerosis, Parkinson's disease, and Alzheimer's disease. A specific group of interesting diseases and disorders includes Diabetes Mellitus Type I Rheumatoid Arthritis, Systemic Lupus Erythematosus, Chronic lymphocytic thyroiditis, Multiple Sclerosis and Ulcerative Colitis.

Alternatively it may be used for the manufacturing of a medicament to be used in transplantations to inhibit immune rejection of organs, tissues, normal or gene therapeutically modified cells

### BRIEF DESCRIPTION OF THE DRAWINGS

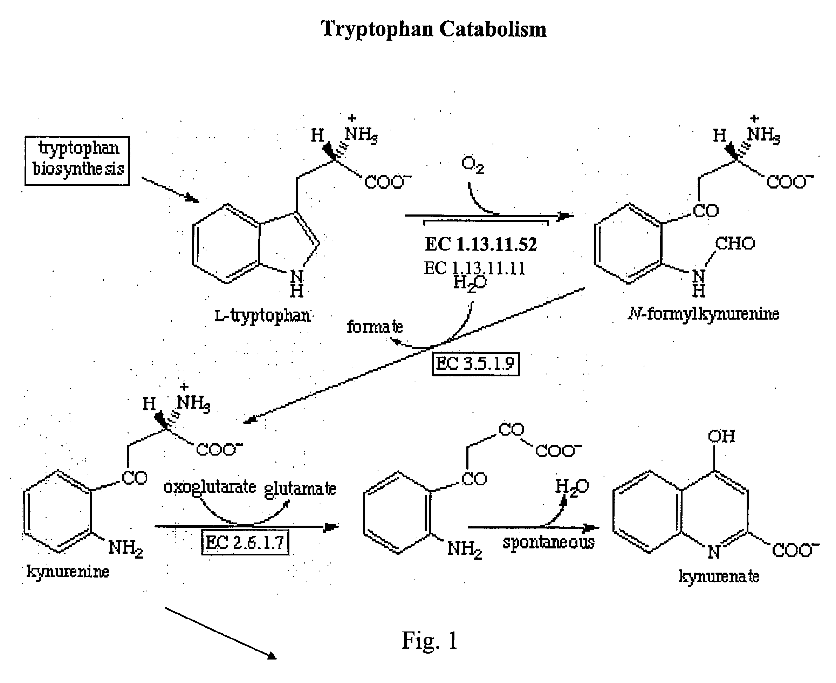

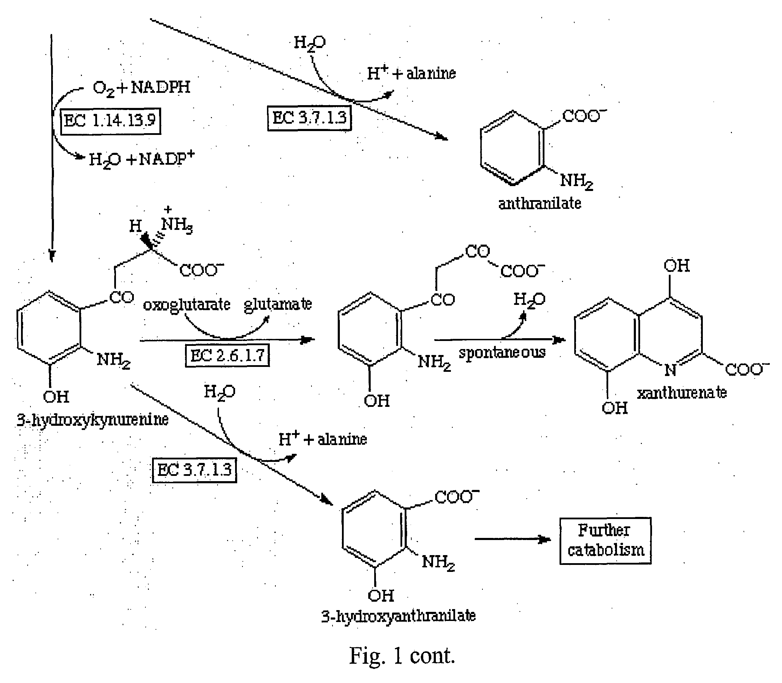

Fig 1 shows the tryptophan catabolism, EC 1.13.11.11 is tryptophan 2,3-dioxygenase, EC 1.13.11.52 is indoleamine 2,3-dioxygenase (IDO), EC 1.14.13.9 is kynurenine 3-monooxygenase, EC 2.6.1.7 is kynurenineoxoglutarate transaminase, EC 3.5.1.9 is arylformamidase and EC 3.7.1.3 is kynureninase.
Fig 2 shows the chemical structure of zebularine.
Fig 3 shows qRT-PCR analysis of the IDO mRNA expression levels in H1D2WT, H1D2IL12C46, and H1D2IL18C2 rat colon cancer cell lines untreated or treated with zebularine at 20 µM and 100µM of zebularine respectively. The values given are normalized in relation to the HPRT expression values.
Fig 4 shows zebularine treated lymphocytes. Fig 4A show proliferation of stimulated human lymphocytes after five days in culture in the presence of zebularine at different concentrations, fig 4B show restoration of inhibited proliferation of stimulated human lymphocytes after five days in culture in the presence of zebularine at two different concentrations and the IDO inhibitor 1-methyl tryptophane at 100 µM and fig 4C show proliferation of stimulated rat spleen lymphocytes after four days in culture in the presence of zebularine at different concentrations.

### DETAILED DESCRIPTION OF THE INVENTION

In the context of the present application and invention, the following definitions apply:
The term "immunoprotective" is defined herein as an effect which reduces, arrests, or ameliorates immunological insult and is protective, resuscitative or revivative for affected tissue that has suffered cytotoxic insult from immune cells or inflammation.
The term "immunoprotective agent" is herein defined as active ingredient or medicament containing an immune insult treatment dose of active ingredient effective in reducing, preventing, arresting, or ameliorating immune insult and provides protection, resuscitation or revival to affected tissue that has suffered immune mediated insult.
The term "indolamine dioxygenase (IDO)" is intended to mean IDO-1 (indoleamine 2,3-dioxygenase, EC 1.13.11.52), IDO-2 (indoleamine-pyrrole 2,3 dioxygenase-like 1, EC 1.13.11.-) or TDO (tryptophan 2,3-dioxygenase, EC 1.13.11.11) that are three different proteins that can catabolize tryptophan. IDO-1 can also catabolize serotonin and melatonin although the substrate specificity for IDO-2 and TDO is not so well studied. Metabolites or catabolites from the tryptophan pathway are Tryptophan, N-Formyl-kynurenine, Formylanthranilate, Anthranilate, L-Kynurenine, 4-(2-Aminophenyl)-2,4-dioxybutanoate, Kynurenic acid, 3-Hydroxy-L-kynurenine, 3-Hydroxy-anthranilate, 3-Metoxy-anthranilate, 4-(2-Amino-3-hydroxy-phenyl)-2,4-dioxobutanoate, Xanthurenate, 8-Metoxy-kurenate, 2-Amino-3-carboxy-muconate semialdehyde, 2-Aminomuconate semialdehyde, Quimolinic acid, Cinnavalininate, Tryptamine, N-Methyltryptamine, Indoleacetate, 2-Formamino-benzoylacetate, 5-Hydroxy-L-tryptophan, 5-Hydroxy-N-formylkunerine, 5-Hydroxy-kunerine, 5-Hydroxy-kunerenamin, 4,6-Dihydroxy-quinoline, Serotonin, N-Acetyl-serotonin, Melatonin, 6-Hydroxy-melatonin, Formyl-N-acetyl-5-metoxykynurenamine, N-Methylserotonin, Formyl-5-hydroxy-kynurenamine, 5-Metoxytryptamine, 5-Hydroxyindole-acetaldehyde, 5-Hydroxyindoleacetate, 5-Metoxyindoleacetate, or 5-Hydroxyindole-acetylglycine to enhance the immunosuppressive IDO activity. Examples are Kynurenine, 3-hydroxy-kynurenine, anthranilic acid, 3-hydroxy-anthranilic acid, quinolinic acid and picolinic acid. Also enhancements using synthetic variants of tryptophan catabolites, e.g., N-(3,4,-Dimethoxycinnamoyl) anthranilic acid.

The immune suppression mediated by IDO is mediated by starvation of Tryptophan, induction of apoptosis in lymphocytes and induction of regulatory T lymphocytes (Treg). Hence, the apoptosis induction and Treg induction is mediated by the catabolites, why addition of such catabolites in combination with IDO induction by medicament of the invention may enhance the clinical effect. The immune suppressive action from IDO-1, IDO-2 and TDO may be explained by 1) starvation of tryptophan, 2) direct toxic effect from several of the above mentioned metabolites/catabolites that induce apoptosis of immune cells, particularly L-Kynurenine, Anthranilate, 3-Hydroxy-anthranilate and 3-Hydroxy-L-kynurenine and 3) that some of the metabolites/catabolites stimulate the differentiation of T helper cells to immune suppressive regulatory T-cells important for tolerance.

An analogue is a molecule that differs in chemical structure from a parent compound, for example a homolog (differing by an increment in the chemical structure, such as a difference in the length of an alkyl chain), a molecular fragment, a structure that differs by one or more functional groups, a change in ionization. Structural analogues are often found using quantitative structure activity relationships (QSAR), with techniques such as those disclosed in Remington (The Science and Practice of Pharmacology, 19th Edition (1995), chapter 28). A derivative is a substance related to a base structure, and theoretically derivable from the base structure. A mimetic is a biomolecule that mimics the activity of another biologically active molecule. Biologically active molecules can include chemical structures that mimic the biological activities of a compound, for instance zebularine.

The IDO gene expression is known to be induced in antigen presenting cells and is subject to complex regulation by an array of signals. For example, IFN-γ□ can signal through JAK and STAT1 together with the sis-acting IFN-γ-stimulated response elements (ISRE) on the IDO promoter, activating transcription of IDO. However, bacterial lipopolysaccharides (LPS), interleukin-1- beta (IL-1β), and TNF can also enhance IDO expression. Also in human epithelial cells, IFN-γ-induced IDO expression is transcriptionally enhanced by tumour necrosis factor-alpha (TNF-α). It is possible that also an IFN-γ-independent induction mechanism exists.

The invention relates to the use of at least 1-(β-D-Ribofuranosyl)-1,2-dihydropyrimidin-2-one, determined analogues thereof or salts thereof for the manufacturing of a medicament for the treatment of an autoimmune disorder or disease or immune rejection of transplants or gene therapeutically modified cells as listed above, wherein the treatment induces indolamine dioxygenase. Said composition will be used as an immunoprotective agent.

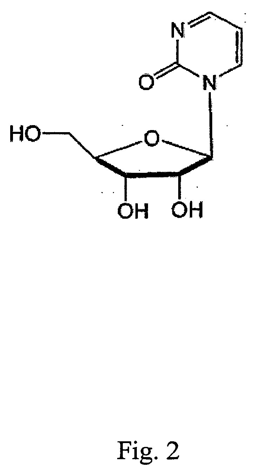

1-(β-D-Ribofuranosyl)-1,2-dihydropyrimidin-2-one also named Zebularine (Fig 2) is a drug that the inventors have shown to strongly enhance the production of IDO in concentrations of 100 µM, which is far below toxicity level.

Zebularine will induce the production of IDO that is a natural immune suppressive molecule. As such, zebularine would help to control unwanted immune reactivity. Moreover it is likely that induction of IDO can lead to the induction of tolerance that would make continuous immunosuppressive therapy unnecessary and in this way avoid associated side effects.

The chemical structure of Zebularine is shown in Fig 2.

Zebularine Synonyms are 1-(β-D-Ribofuranosyl)-1,2-dihydropyrimidin-2-one or 2-Pyrimidone-1-β-D-riboside. Analogues, mimetics and derivatives used according to the invention are 5-methylcytidine, 2'-deoxyzebularine, 5-fluoro-zebularine, 5-fluoro-2'-dexyzebularine, 5-chloro-zebularine, 5-chloro-2'-dexyzebularine, 5-bromo-zebularine, 5-bromo-2'-dexyzebularine, 5-iodo-zebularine, 5-iodo-2'-dexyzebularine, 5-Me-2'-deoxyzebularine, or mono, di or tri phosphates thereof.

Zebularine represents a completely new class of substances for use as immunomodulators for immunosuppression and tolerance induction. Zebularine is an immunoprotective agent at acceptable physiologic and pharmacologic doses.

The invention is the use of for example zebularine for obtaining a treatment medicine and medicament intended for the therapeutic immunoprotective use of treating autoimmune disease and patients receiving allotransplants or undergoing gene therapy with associated risks of rejection of cells expressing the transferred genes. Examples of other diseases are found above.

The medicament, such as zebularine is suited for the immunoprotective treatment of cell cultures, in vitro treatment of transplants, transfected, genetically engineered cell cultures or transplants.

The medicament may be administrated to a mammal in need thereof in a suitable amount to achieve an effect corresponding to such concentrations that induce a strong IDO activity in vitro of said methyl transferase inhibitor alone or in combination with other inducers of IDO. Other inducers of IDO can be free fatty acids, interferons, toll-like receptor ligands, histone deactylase inhibitors (HDACi).

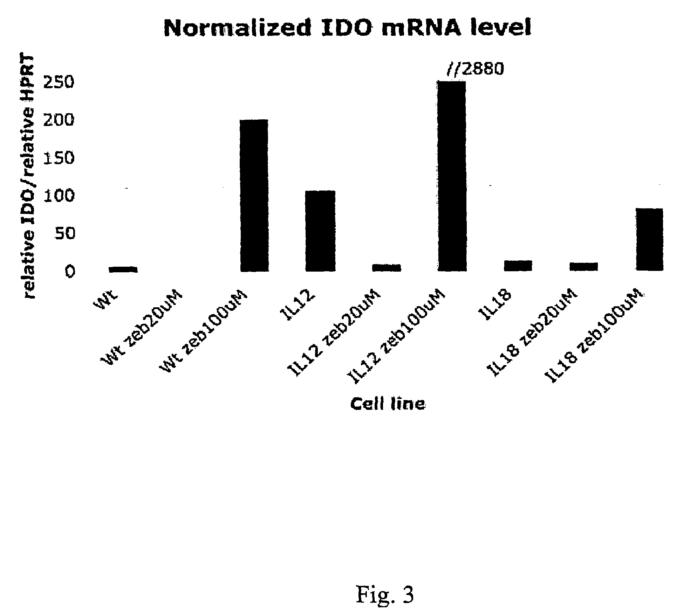

The inventors investigated how zebularine, a methyl transferase inhibitor, affected the IDO production from three rat colon cancer cell lines (Fig. 3) , and found that the IDO expression dramatically increased at a zebularine concentration of 100 µM. The treatment with 100 µM of zebularine induced IDO mRNA expression in all three cell lines. A 48-fold increase of the IDO mRNA level in the H1D2WT cell line, a 24-fold increase in the H1D2IL12C46 cell line and a 14-fold increase in the H1D2IL18C2 cell line were detected. Immunization of rats with tumour cells pretreated with 100 µM of zebularine also resulted in a very weak proliferative response of non-adherent spleen cells stimulated with tumor cells in vitro. The inventors know that the 100 µM treatment of tumour cells strongly induces the production of the immunosuppressive molecule IDO but the induction of other T cell immunosuppressive molecules might also be involved, although no evidence has been found for an effect on prostaglandine E2 (PGE2) or nitric oxide (NO) production. It seems that the 100 µM zebularine treatments are not affecting the PGE2 production, the NO production, or the production of tumour antigens.

By having a direct effect on the immune system, treatment with a dose corresponding to an in vitro dose of 5 to 1000 µM zebularine, alone or in combination with other inducers of IDO such as free fatty acids, interferons, toll-like receptor ligands or histone deactylase inhibitors (HDACi) can be used for pretreatment of transplants (organs, tissues or cells) inducing IDO expression in the endothelial cells and as a consequeence making them less immunogenic to the host and reducing the risk of rejection of the grafted cells. By subsequent treatment of the graft recipients with zebularine at a dose providing immune suppression in vivo and induction of immunological tolerance permanent survival of the transplants can be achieved without further therapy or with minimal such therapy. Similar treatment can be used to control and even cure autoimmune diseases, such as arthritis, MS and diabetes type I by inducing tolerance to the tissue specific antigens involved.

Zebularine is highly hydrophilic, and soluble in water. Zebularine may be administered orally or intravenously (i.v.) or by any other suitable route. In the mouse, the bioavailability after i.v. injection is good and about 60% of a dose of 100 mg/kg is absorbed in tissues like plasma, red blood cells, liver, kidney, spleen, heart, lung, muscle, Duodenum, Jejunum, Ileum, Colon and Carcass. The bioavailability for Fat, Brain, Testes, Cecum and Stomach is less. The peak concentrations are reached 5 to 10 minutes after administration and the turnover is quite fast for many tissues. The bioavailability has been compared to that of uridine and found to be very similar.

Zebularine is in the mouse mainly metabolized to uridine, uracil and dihydrouracil.

When a patient is to be treated by the invented medicament such as zebularine it may be administrated in an amount which varies between 5 um to 200 uM, depending on which disease or disorder to be treated. Initially a higher dose may be used such as 75 to 1000 uM or 100 uM followed by a maintaining dose of 5-65 uM, such as 50 uM.

The invented medicament may further comprise a pharmaceutically acceptable buffer, excipient, diluent or carrier.

"Pharmaceutically acceptable" means a non-toxic material that does not decrease the effectiveness of the biological activity of the active ingredients.. Such pharmaceutically acceptable buffers, carriers or excipients are well-known in the art (see Remington's Pharmaceutical Sciences, 18th edition, A.R Gennaro, Ed., Mack Publishing Company (1990) and handbook of Pharmaceutical Excipients, 3rd edition, A. Kibbe, Ed ., Pharmaceutical Press (2000).

The term "buffer" is intended to mean an aqueous solution containing an acid-base mixture with the purpose of stabilising pH. Examples of buffers are Trizma, Bicine, Tricine, MOPS, MOPSO, MOBS, Tris, Hepes, HEPBS, MES, phosphate, carbonate, acetate, citrate, glycolate, lactate, borate, ACES, ADA, tartrate, AMP, AMPD, AMPSO, BES, CABS, cacodylate, CHES, DIPSO, EPPS, ethanolamine, glycine, HEPPSO, imidazole, imidazolelactic acid, PIPES, SSC, SSPE, POPSO, TAPS, TABS, TAPSO and TES.

The term "diluent" is intended to mean an aqueous or non-aqueous solution with the purpose of diluting the medicament. The diluent may be one or more of saline, water, polyethylene glycol, propylene glycol, ethanol or oils (such as safflower oil, corn oil, peanut oil, cottonseed oil or sesame oil).

The excipient may be one or more of carbohydrates, polymers, lipids and minerals. Examples of carbohydrates include lactose, sucrose, mannitol, and cyclodextrines, which are added to the composition, e.g." for facilitating lyophilisation. Examples of polymers are starch, cellulose ethers, cellulose carboxymethylcellulose, hydroxypropylmethyl cellulose, hydroxyethyl cellulose, ethylhydroxyethyl cellulose, alginates, carageenans, hyaluronic acid and derivatives thereof, polyacrylic acid, polysulphonate, polyethylenglycol/polyethylene oxide, polyethyleneoxide/polypropylene oxide copolymers, polyvinylalcohol/polyvinylacetate of different degree of hydrolysis, and polyvinylpyrrolidone, all of different molecular weight, which are added to the composition, e.g., for viscosity control, for achieving bioadhesion, or for protecting the lipid from chemical and proteolytic degradation. Examples of lipids are fatty acids, phospholipids, mono-, di-, and triglycerides, ceramides, sphingolipids and glycolipids, all of different acyl chain lenght and saturation, egg lecithin, soy lecithin, hydrogenated egg and soy lecithin, which are added to the composition for reasons similar to those for polymers. Examples of minerals are talc, magnesium oxide, zinc oxide and titanium oxide, which are added to the composition to obtain benefits such as reduction of liquid accumulation or advantageous pigment properties.

The invented medicament may be administrated by any suitable route including oral, sublingual, buccal, nasal, inhalation, parenteral (including intraperitoneal, intraorgan, subcutaneous, intradermal, intramuscular, intra-articular, venous (central, hepatic or peripheral), lymphatic, cardiac, arterial, including selective or superselective cerebral arterial approach, retrograde perfusion through cerebral venous system, via catheter into the brain parenchyma or ventricles), direct exposure or under pressure onto or through the brain or spinal tissue, or any of the cerebrospinal fluid ventricles, injections into the subarachnoid, brain cisternal, subdural or epidural spaces, via brain cisterns or lumbar puncture, intra and periocular instillation including application by injection around the eye, within the eyeball, its structures and layers, the ear, including the Eustachian tube, mastoid air cells, external and internal auditory canals, tympanic membrane, middle ear, inner ear including the cochlear spiral ganglion and labyrinthine organs, as well as via enteral, bowel, rectal, vaginal, urethral or bladder cisternal. Also for in utero and perinatal indications then injections into the maternal vasculature, or through or into maternal organs including the uterus, cervix and vagina, and into embryo, foetus, neonate and allied tissues and spaces such as the amniotic sac, the umbilical cord, the umbilical artery or veins and the placenta, with parenteral being the preferred route. The preferred route may vary depending on the condition of the patient.

The effect of the invented medicament may be further potentiated not only by combining it with other IDO stimulating drugs as mentioned above but also in combination with an immunosuppressive agent to reduce the frequency of effector immune cells during or before the stimulation of tolerance.

This invention includes the possibility of the timing and sequence of delivery of active ingredients to induce tolerance. In order to have the best opportunity to protect tissue from immune mediated insult, the medicament needs to be available as soon as possible within the cells of the graft. This would induce IDO expression in endothelial cells, which by itself suppresses rejection responses.

The medicament may comprise additional active ingredients such as glycocorticoids, methotrexate, rapamycin, cyclophosphamide, antimetabolites including azathioprine, immunophilin-binding drugs (including tolerance, tacrolimus, sirolimus, everolimus), inhibitors of nucleotide synthesis (including mycophenolate mofetil, mizoribine, leflunomide, FK778), FTY720, lymphocyte depleting antibodies (including polyclonal antibodies to lymphocytes, thymocytes, T-cells, muromonab-CD3, rituximab, Alemtuzumab, CAMPATH-1), non-depleting antibodies (including daclizumab, basiliximab, the two CTLA-4-Ig fusion proteins LEA29Y and abatacept, LFA3-Ig fusion protein), anti-TNF antibodies (including infliximab, adalimumab), natalizumab (anti-VLA-4), the anti-CD 154 antibodies BG9588 and IDEC 131), soluble cytokine receptors (including lenercept and etanercept (soluble TNF p55 and TNF p75 receptors), histone deacetylase inhibitors and anakinra (soluble IL-1RA).
Other examples includes transfer of genes such as arginase-1, or substances like prostaglandin E2 (PGE2), cyclosporine A.

These immune suppressive drugs mentioned above can be used in combination with the medicament of the invention to reduce the number of immune cells.

The bioavailability of the medicament such as zebularine is partly dependent on the activity of aldehyde oxidase that can add water to the position 4 of zebularine and thereby converting zebularine into uridine. The activity of aldehyde oxidase is high in the liver. There exist a large number of aldehyde oxidase inhibitors (see Obach et al., 2004) and inhibitors with IC₅₀ values from 3 nM up to 1 µM are e.g., Raloxifene, Perphenazine, Thioridazine, Menadione, Trifluperazine, Amitriptyline, Estradiol, Felodipine, Clomipramine, Loratidine, Promethazine, Chlorpromazine, Ethinyl estradiol, Norclomipramine, Amodiaquine, Nortriptylin.

The medicament includes those suitable for administration by the routes including oral, sublingual, buccal, nasal, inhalation, parenteral (including intraperitoneal, intraorgan, subcutaneous, intradermal, intramuscular, intra-articular, venous (central, hepatic or peripheral), lymphatic, cardiac, arterial, including selective or superselective cerebral arterial approach, retrograde perfusion through cerebral venous system, via catheter into the brain parenchyma or ventricles), direct exposure or under pressure onto or through the brain or spinal tissue, or any of the cerebrospinal fluid ventricles, injections into the subarachnoid, brain cisternal, subdural or epidural spaces, via brain cisterns or lumbar puncture, intra and periocular instillation including application by injection around the eye, within the eyeball, its structures and layers, the ear, including the Eustachian tube, mastoid air cells, external and internal auditory canals, tympanic membrane, middle ear, inner ear including the cochlear spiral ganglion and labyrinthine organs, as well as via enteral, bowel, rectal, vaginal, urethral or bladder cisternal. Also for in utero and perinatal indications then injections into the maternal vasculature, or through or into maternal organs including the uterus, cervix and vagina, and into embryo, fetus, neonate and allied tissues and spaces such as the amniotic sac, the umbilical cord, the umbilical artery or veins and the placenta, with parenteral being the preferred route.

The medicament may be distributed and made available in convenient unit dose form such as capsules and ampoules, containing the active ingredient of the invention, and may be manufactured and distributed by any of the methods known to the pharmaceutical arts. In addition to the active ingredient, the medicament can also contain other usual agents of the art relating to the type of medicament produced. This may, by example, take the configuration of suspensions, solutions and emulsions of the active ingredient in lipid, non-aqueous or aqueous diluents, solvents, dissolving agents, emulsifiers, syrups, granulates or powders, or mixtures of these. The medicament can also contain colouring agents, preservatives, perfumes, flavouring additions and sweetening agents. In addition to the active ingredient, the medicament can also contain other pharmaceutically active medications. The manufacture and distribution of the medicament is carried out by techniques known to the art, such as, evenly and intimately bringing together the active ingredient with liquids or fine solids or both, and then if needed, forming the medicament into a dose unit form. The discrete dose, portion and carrier vehicle constituting the medicament will generally be adapted by virtue of shape or packaging for medical administration and distributed for this purpose.

Tablets can be manufactured and distributed by compression or mould, from active ingredient possibly with one or more additional pharmaceutically active compounds. Compressed tablets can be manufactured and distributed through compression in a machine typical to the art a known quantity of the active ingredient in a dispersible configuration such as powder or granules, possibly mixed with other agents including binders, lubricants, inert diluents, preservatives, and dispersing agents. Moulded tablets can be manufactured and distributed by moulding in a machine typical to the art a mix of known quantity of active ingredient addition pharmaceutically active compounds and other additives moistened with a liquid diluent. The tablets can possibly be coated, enveloped or covered, with substances including protective matrices, which can contain opacifiers or sweeteners and can be formulated to allow slow or controlled release, or also release within a certain part of the digestive system of the contained active ingredients. Capsules can be manufactured and distributed by placement of a known quantity of active ingredient, additional pharmaceutically active compounds and additives within a two part or sealed capsule of gelatine or other aqueous dissolvable substance. The active ingredient can also be manufactured and distributed as a medicament in microencapsulated, microsomal, micellar and microemulsion forms.

The medicament containing the active ingredient acceptable for oral topical administration can be manufactured and distributed as lozenges containing the active ingredients, other pharmaceutically active compounds, and additives in a flavoured basis, such as acacia and tragacanth; Pastilles containing the active ingredient with other pharmaceutically active compounds, and additives in an inert base such as gelatine and sucrose: Mouthwashes or rinses containing the active ingredient with other pharmaceutically active compounds, and additives in an acceptable liquid.

The medicament containing the active ingredient acceptable for skin topical administration can be manufactured and distributed as ointments, oils, creams, lotions, gels, pastes and transdermal patch containing the active ingredient, other pharmaceutically active compounds, additives and an acceptable carrier medium.

The medicament containing the active ingredient acceptable for nasal administration can be manufactured and distributed with other pharmaceutically active compounds and additives as a powder for inhalation, or as an oily, aqueous or non-aqueous liquid for nasal spray or drops.

The medicament containing the active ingredient acceptable for rectal administration can be manufactured and distributed as suppositories, creams, foams, douches or enemas with other pharmaceutically active compounds, suitable bases of the usual water-soluble diluents, fats, and additives known to practitioners of the art.

The medicament containing the active ingredient acceptable for vaginal administration can be manufactured and distributed as pessaries, suppositories, creams, gels, foams, douches or sprays with other pharmaceutically active compounds, suitable bases and additives known to practitioners of the art.

The medicament containing the active ingredient acceptable for parenteral administration can be manufactured and distributed from aqueous and non-aqueous sterile injection solutions, other pharmaceutically active compounds, additives including anti-oxidants, bacteriostats and solutes and sugars such as mannitol to make the medicament isotonic, hypotonic or hypertonic with the blood of the recipient; and also aqueous and non-aqueous sterile suspensions which can include suspenders and thickeners. The medicament can be manufactured and distributed in unit-dose or multi-dose containers, such as sealed glass or plastic ampoules, vials, bottles and bags as a liquid, and in a dry state requiring only the addition of sterile liquid, for example water, saline or dextrose solutions, immediately prior to use. Extemporaneous solutions and suspensions for injection can be prepared from powders and tablets of the kind above described.

The medicament containing the active ingredient acceptable for administration into the brain and related structures, spinal cord and related structures, ventricular system and cerebrospinal fluid spaces can be manufactured and distributed from aqueous and non-aqueous sterile injection solutions, other pharmaceutically active compounds, additives including anti-oxidants, bacteriostats and solutes and sugars such as mannitol to make the medicament isotonic, hypotonic or hypertonic with the cerebrospinal fluid; and also aqueous and non-aqueous sterile suspensions which can include suspenders and thickeners. The medicament can be manufactured and distributed in unit-dose or multi-dose containers, such as sealed glass or plastic ampoules, vials, bottles and bags as a liquid, and in a dry state requiring only the addition of sterile liquid, for example water, saline or dextrose solutions, immediately prior to use. Extemporaneous solutions and suspensions for injection can be prepared from powders and tablets of the kind above described.

The desired unit dose of medicaments, are those containing a daily dose or immune insult treatment dose or an appropriate fraction thereof, of the administered active ingredient. Unit dose forms of the invention may also include more complex systems such as double barrelled syringes, syringes with sequential compartments one of which may contain the active ingredient, and the other any necessary diluents or vehicles. The agents in the syringes would be released sequentially or as a mixture or combination of the two after the triggering of the syringe plunger. Such systems are known in the art.

The medicament may be used for the treatment of a disease or disorder such as those mentioned above.

Following examples are intended to illustrate, but not to limit, the invention in any manner, shape, or form, either explicitly or implicitly.

### EXAMPLES

Example 1: Organs prepared for transplantation was perfused with cold physiological salt saline (or sodium chloride) solution containing Zebularine at a concentration of 100 µM and incubated in that solution until the surgical transplantation procedure was performed. One day prior to transplantation, the recipient patient is treated with oral Zebularine at an optimal dose for induction of IDO at an immunosuppressive concentration and oral cyclosporine at standard dosage continued daily for 2 weeks and at a reduced dose level for another 2 weeks. During the 4 first days after surgery dexamethasone is administered intravenously at a daily dose of 8 mg and the dose is then tapered over the following 2 weeks. One month after transplantation cyclosporine treatment is stopped, whereas the Zebularine is continued and patients are closely watched for evidence of rejection. After 2 months of treatment with Zebularine alone this treatment is stopped provided nonresponsiveness to donor but not third part cells can be demonstrated in vitro.

Example 2: A patient with a bilateral severe ocular-surface disorder is to receive allogeneic corneal epithelial stem-cell transplantation (as reported by Tsubota et al, NEJM, 340,1697-1703, 1999). One day prior to transplantation the recipient patient is treated with oral Zebularine at an optimal dose for induction of IDO at an immunosuppressive concentration and oral cyclosporine at standard dosage continued daily for 2 weeks and at a reduced dose level for another 2 weeks. During the 4 first days after surgery dexamethasone is administered intravenously at a daily dose of 8 mg and the dose is then tapered over the following 2 weeks. One month after transplantation cyclosporine treatment is stopped, whereas the Zebularine is continued and patients closely watched for evidence of rejection. After 2 months of treatment with Zebularine alone this treatment is stopped provided nonresponsiveness to donor but not third part cells can be demonstrated in vitro.

Example 3: A patient suffering from critical limb ischemia is subjected to gene therapy with direct intramuscular administration of the eukaryotic expression vector pUC18 encoding VEGF₁₆₅ transcriptionally regulated by the cytomegalovirus promoter/enhancer in a total of 4 mg of DNA in 8 aliquots into the ischemic limb as previously described (Kalka et al., CIRC RES 2000; 86:1198-1202). The day before gene transfer the patient receives oral Zebularine at an optimal dose for induction of IDO at an immunosuppressive concentration and this treatment is continued for 3 months by oral administration in order to prolong the action of the VEGF by protecting against the immune rejection of the muscle cells expressing the vector-encoded proteins. Plasma VEGF levels are measured by an ELISA assay to monitor the maintained expression of the transferred VEGF gene.

Example 4: A patient with active Rheumatoid Arthritis with considerable remaining symptoms despite treatment with Methotrexate 10 mg per week receives oral Zebularine at an optimal dose for induction of IDO at an immunosuppressive concentration. Methotrexate therapy is continued for 6 weeks. Zebularine therapy is then continued as single therapy for 3 months in order to induce immunosuppression and immunological tolerance.

Example 5: A patient with a diagnosis of relapsing-remitting multiple sclerosis, a score on the Expanded Disability Status Scale of 4 (on a range of 0-10), a MRI scan revealing lesions consistent with a diagnosis of multiple sclerosis, and having had a relapse despite having received treatment with interferon beta-1a for more than 12 months at a dose of 30 microgram i.m. per week. At maintained interferon beta-1a therapy oral Zebularine therapy is initiated at an optimal dose for induction of IDO at an immunosuppressive concentration. The interferon beta-1a therapy is stopped after 3 weeks and Zebularine therapy continued for 12 months and then stopped provided that no relapse has occurred.

Example 6: A young patient with the diagnosis Diabetes Mellitus type I in an early stage is treated with insulin and within 3 weeks of diagnosis with oral Zebularine at an optimal dose for induction of IDO at an immunosuppressive concentration. The required insulin dose is closely monitored beyond the expected initial decrease until it has stabilized at a low level or is no longer required. Zebularine therapy is maintained for 4 months and then stopped. Resumed Zebularine therapy dependent on a close monitoring of signs of increased requirement of insulin.

Example 7: Immune cells treated with zebularine in vitro (The use of in vitro treatment of immune cells with zebularine) can be utilized for transplantation. Peripheral blood from a patient with diabetes type I who is to receive a (that will be given) pancreatic beta cell transplant (transplantation) is collected. White blood cells from the patient blood are isolated and cultured in the presence of GMCSF, protein extract from the donor and zebularine. The cells are then stimulated with CpG-containing oligonucleotides and after further culture in vitro in presence of zebularine and HDAC inhibitors, regulatory plasmacytoid dendritic cells are enriched and are given back to the patient. (The in vitro treatment can also be combined with e.g., treatment with CTLA4-Ig or HDAC inhibitors.) These regulatory DC will then present antigens from the donor and induce tolerance against these foreign antigens. The beta cells from the donor are treated in vitro with zebularine prior to tranplantation. The patient can be given a pretreatment with zebularine, alone or in combination with HDAC-inhibitors, rapamycin, CTLA4-Ig, alone or in combination). The in vivo treatment will continue for about 2 weeks after transplantation of the pancreatic, insulin producing beta-cells. The patient is monitored by FACS analysis for presence of FoxP3 regulatory T-cells having T-cell receptors that can bind beta cell peptides conjugated with soluble fluorescent HLA-DR complexes. (The analysis is followed using a fluorescence activated cell sorter (FACS).) When an increased frequency (frequence) of regulatory T-cells can be identified, the immune suppressive treatment will be reduced until the patient does not need immune suppression and is cured.

Example 8: Immune cells treated with zebularine in vitro (The use of in vitro treatment of immune cells with zebularine) can be utilized in patients with autoimmune disease (for autoimmunity). Peripheral blood from a patient with reumatoid arthritis is collected. White blood cells from the patient blood are isolated and cultured in the presence of GMCSF, collagen or cartilage and zebularine. The cells are then stimulated with CpG-containing oligonucleotides and after further culture in vitro in presence of zebularine and HDAC inhibitors, regulatory plasmacytoid dendritic cells are enriched and are given back to the patient. (The in vitro treatment can also be combined with e.g., treatment with CTLA4-Ig or HDAC inhibitors.) These regulatory DC will then present antigens from collagen and reinduce (induce) tolerance against these self antigens (that is broken) in the RA patient. The patient can be given a pretreatment with zebularine, alone or in combination with HDAC-inhibitors, rapamycin, CTLA4-Ig (alone or in combination). In vivo treatment will continue for about 2 weeks after injection of the in vitro treated tolerogenic DC cells. The patient is monitored by FACS analysis for presence of FoxP3 regulatory T-cells having T-cell receptors that can bind collagen peptides conjugated with soluble fluorescent HLA-DR complexes. The patient is also monitored for the presence of CD8+ effector T-cells with TcR that can bind soluble fluorescent HLA-A proteins conjugated with collagen peptides. (The analysis is followed using a fluorescence activated cell sorter (FACS).) When an increased frequency (frequence) of regulatory T-cells and a significant decrease in CD8+ effector cells can be identified, the immune suppressive treatment will be reduced until the patient does not need immune suppression and is cured, i.e., the natural tolerance has been restored.

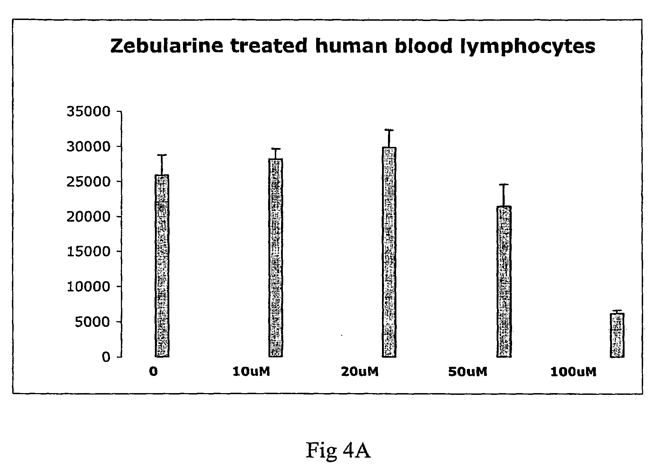

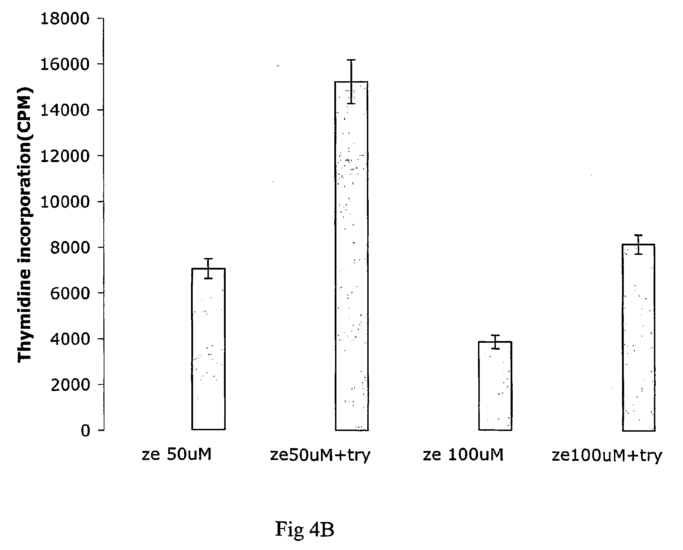

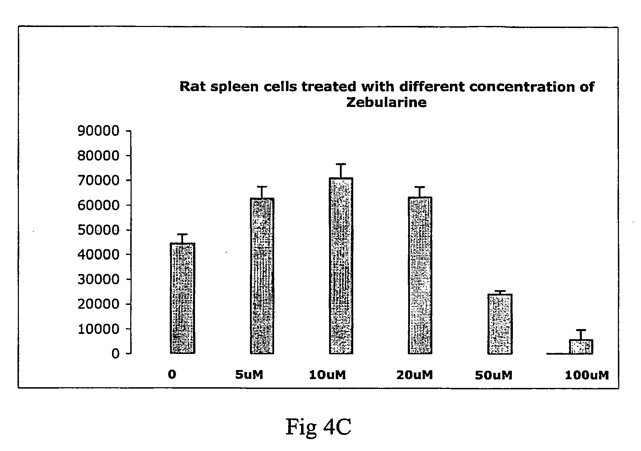

Example 9: In an experiment the inventors tested the immunogenicity of genetically engineered rat colon cancer cells expressing rat interleukin 12. These cancer cells have repeteadly been used to immunize rats that create a strong immune response towards the rat tumour cells. The inventors pre-treated these rat colon cancer cells with 100 µM zebularine that rendered the rat colon cancer cells less immunogenic when used for immunization. This was congruent with the changes in expression of IDO (Fig. 3). Similar treatments of rat spleen cells and human buffy coat leukocytes induce enhanced IDO production and strong suppression of polyclonal lymphocyte proliferation, i.e. suppression of T cell activation. There is a very reproducible induction of IDO (about 20-fold) from different human leukocyte cultures with a slight interindividual variation. The suppression of proliferation can be blocked by addition of the IDO inhibitor 1-methyl tryptophan (1-MT) as can be seen from figure 4.

### References

Cheng, J.C. , C. B. Matsen, F.A. Gonzales, W. Ye, S. Greer, V.E. Marquez, et al., Inhibition of DNA methylation and reactivation of silenced genes by Zebularineularine, Journal of the National Cancer Institute 95 (2003) 399-409.
Cheng, J.C. , D. J. Weisenberger, F.A. Gonzales, G. Liang, G. L. Xu, Y.G. Hu, et al., Continuous Zebularineularine treatment effectively sustains demethylation in human bladder cancer cells, Molecular and Cellular Biology 24 (2004) 1270-1278.
Cheng, J.C., C. B. Yoo, D.J. Weisenberger, J. Chuang, C. Wozniak, G. Liang, et al., Preferential response of cancer cells to Zebularineularine, Cancer Cell 6 (2004) 151-158.
Liu, G. , H. Ying, G. Zeng, C. J. Wheeler, K. L. Black, J. S. Yu, HER-2, gp 100, and MAGE-1 are expressed in human glioblastoma and recognized by cytotoxic T cells, Cancer Research, 64 (2004) 4980-4986.
Munn, D.H., M.D. Sharma, D. Hou, B. Baban, J.R.Lee, S.J. Antonia, et al., Expression of indoleamine 2,3-dioxygenase by plasmacytoid dendritic cells in draining-draining lymph nodes. The Journal of Clinical Investigation, 114 (2004) 280-290.
Obach RS., Huynh P., Allen M.C., and Beedham C. Human Liver Aldehyde Oxidase: Inhibition by 239 Drugs. Journal of Clinical Pharmacology, 2004; 44:7-19
Miki T, Sun H, Lee Y, Tandin A, Kovscek AM, Subbotin V, Fung JJ, Valdivia LA. Blockade of tryptophan catabolism prevents spontaneous tolerogenicity of liver allografts. 2001 Transplantation Proceedings, Volume 33, Issue 1-2, Pages 129-130
Mellor, A.L., D.H. Munn, IDO expression by dendritic cells: tolerance and tryptophan catabolism, Nature Reviews Immunology 4 (2004) 762-774.
Uyttenhove, C. , L. Pilotte, I. Theate, V. Stroobant, D. Colau, N. Parmentier, et al., Evidence for a tumoral immune resistance mechanism based on tryptophan degradation by indoleamine 2,3-dioxygenase, Nature Medicine 9 (2003) 1269-1274.

## Claims

1. Use of 1-(β-D-Ribofuranosyl)-1,2-dihydropyrimidin-2-one or salts thereof or an analogue, wherein said analogue is selected from the group consisting of 5methylcytidine, , 2'-deoxyzebularine, 5-fluoro-zebularine, 5-fluoro-2'-dexyzebularine, 5-chloro-zebularine, 5-chloro-2'-dexyzebularine, 5-bromo-zebularine, 5-bromo-2'-dexyzebularine, 5-iodo-zebularine, 5-iodo-2'-dexyzebularine, 5-Me-2'-deoxyzebularine, or mono, di or tri phosphates thereof for the manufacturing of a medicament for the treatment of an autoimmune disorder or disease selected from the group consisting of Achlorhydria, Acute hemorrhagic leukencephalitis, Addison's Disease, Alopecia Areata, Anemia, Pernicious Anti - Glomerular Basement Membrane Disease, Antiphospholipid Syndrome, Aplastic Anemia, Atopic Allergy, Autoimmune Atrophic Gastritis, Autoimmune Hearing Loss, Autoimmune hemolytic anemia, Autoimmune hypoparathyroidism, Autoimmune hypophysitis, Autoimmune Lymphoproliferative, Autoimmune Myocarditis, Autoimmune oophoritis, Autoimmune orchitis, Autoimmune Polyendocrinopathy-Candidiasis-Ectodermal-Dystrophy, Autoimmune Syndrome Type II, Polyglandular, Behcet Syndrome, Celiac Disease, Chagas Disease, Cholangitis, Sclerosing, Chronic Inflammatory Demyelinating Polyneuropathy, Chronic lymphocytic thyroiditis, Churg - Strauss Syndrome, Colitis, Ulcerative, Crohn's disease, Cryoglobulinemia, Cushing Syndrome, Dermatitis Herpetiformis, Dermatomyositis, Diabetes Mellitus (Insulin - Dependent), Diffuse Cerebral Sclerosis of Schilder, Encephalomyelitis, Autoimmune, Experimental (EAE), Epidermolysis Bullosa Acquisita, Erythematosis, Felty's Syndrome, Glomerulonephritis (IGA), Glomerulonephritis Membranous, Goodpasture Syndrome, Graves' Disease, Guillain - Barre Syndrome, Hamman-Rich syndrome, Hepatitis Autoimmune, Hepatitis Chronic Active, Idiopathic thrombocytopenia, Inflammatory Bowel Diseases, Insulin resistance - type B, Lambert - Eaton Myasthenic Syndrome, Lens-induced uveitis, Lichen Sclerosus et Atrophicus, Lupus Erythematosus Discoid, Lupus Erythematosus Systemic, Lupus Hepatitis, Lupus Nephritis, Lymphopenia, Meniere's Disease, Mixed Connective Tissue Disease, Mooren's ulcer, Mucocutaneous Lymph Node Syndrome, Multiple Sclerosis, Myasthenia Gravis, Myelitis Transverse, Myocarditis, Narcolepsy, Neuritis Autoimmune Experimental, Neuromyelitis Optica, Oculovestibuloauditory syndrome, Ophthalmia Sympathetic, Opsoclonus - Myoclonus Syndrome, Pancreatitis, Pemphigoid Bullous, Pemphigus foliaceous, Pemphigus Vulgaris, Polyarteritis Nodosa, Polychondritis Relapsing, Polyendocrinopathies Autoimmune, Polymyalgia Rheumatica, Polyradiculoneuropathy, Primary biliary cirrhosis, Purpura Thrombocytopenic Idiopathic, Raynauds, Reiter Disease, Rheumatic Fever, Rheumatoid Arthritis, Sarcoidosis, Scleroderma, Sjögren's Syndrome, Spondylitis Ankylosing, Stiff - Person Syndrome, Still's Disease Adult Onset, Takayasu's Arteritis, Temporal Arteritis, Thyrotoxicosis, Type B Insulin Resistance, Uveomeningoencephalitic Syndrome, Wegener's Granulomatosis, Vitilig or immune rejection of transplants or gene therapeutically modified cells, wherein the treatment induces indolamine dioxygenase.

2. The use according to claim 1, wherein said medicament comprises a pharmaceutically acceptable buffer, excipient, diluent or carrier.

3. The use according to any of preceding claims, wherein said medicament comprises at least two or more 1-(β-D-Ribofuranosyl)-1,2-dihydropyrimidin-2-one or salts thereof.

4. The use according to any of preceding claims, wherein said medicament comprises one or more additional active ingredient.

5. The use according to any of preceding claims, wherein said medicament further comprises at least one compound selected from the group consisting of metabolites of tryptophan, immuno suppressive agents, HDAC inhibitors and substances to increase bioavailability and/or to reduce degradation of 1-(β-D-Ribofuranosyl)-1,2-dihydropyrimidin-2-one.

6. The use according to claim 5, wherein said compound is selected from the group consisting of aldehyde oxidase inhibitors selected from the group consisting of Raloxifene, Perphenazine, Thioridazine, Menadione, Trifluperazine, Amitriptyline, Estradiol, Felodipine, Clomipramine, Loratidine, Promethazine, Chlorpromazine, Ethinyl estradiol, Norclomipramine, Amodiaquine, Nortriptylin to inhibit the oxidation of zebularine to uridine.

7. The use according to claim 5, wherein said compound is selected from the group consisting of Tryptophan, N-Formyl-kynurenine, Formylanthranilate, Anthranilate, L-Kynurenine, 4-(2-Aminophenyl)-2,4-dioxybutanoate, Kynurenic acid, 3-Hydroxy-L-kynurenine, 3-Hydroxy-anthranilate, 3-Metoxy-anthranilate, 4-(2-Amino-3-hydroxy-phenyl)-2,4-dioxobutanoate, Xanthurenate, 8-Metoxy-kurenate, 2-Amino-3-carboxy-muconate semialdehyde, 2-Aminomuconate semialdehyde, Quimolinic acid, Cinnavalininate, Tryptamine, N-Methyltryptamine, Indoleacetate, 2-Formamino-benzoylacetate, 5-Hydroxy-L-tryptophan, 5-Hydroxy-N-formylkunerine, 5-Hydroxy-kunerine, 5-Hydroxy-kunerenamin, 4,6-Dihydroxy-quinoline, Serotonin, N-Acetyl-serotonin, Melatonin, 6-Hydroxy-melatonin, Formyl-N-acetyl-5-metoxykynurenamine, N-Methylserotonin, Formyl-5-hydroxy-kynurenamine, 5-Metoxytryptamine, 5-Hydroxyindole-acetaldehyde, 5-Hydroxyindoleacetate, 5-Metoxyindoleacetate, or 5-Hydroxyindole-acetylglycine.

8. The use according to claim 5, wherein said compound is selected from the group consisting of glycocorticoids, methotrexate, rapamycin, cyclophosphamide, antimetabolites including azathioprine, immunophilin-binding drugs (including tolerance, tacrolimus, sirolimus, everolimus), inhibitors of nucleotide synthesis (including mycophenolate mofetil, mizoribine, leflunomide, FK778), FTY720, lymphocyte depleting antibodies (including polyclonal antibodies to lymphocytes, thymocytes, T-cells, muromonab-CD3, rituximab, Alemtuzumab, CAMPATH-1), non-depleting antibodies (including daclizumab, basiliximab, the two CTLA-4-Ig fusion proteins LEA29Y and abatacept, LFA3-Ig fusion protein), anti-TNF antibodies (including infliximab, adalimumab), natalizumab (anti-VLA-4), the anti-CD154 antibodies BG9588 and IDEC 131), soluble cytokine receptors (including lenercept and etanercept (soluble TNF p55 and TNF p75 receptors), histone deacetylase inhibitors and anakinra (soluble IL-1RA).

9. The use according to any of preceding claims, wherein said medicament is a tablet, a capsule, a solution or a powder.

10. The use according to any of preceding claims, wherein 1-(β-D-Ribofuranosyl)-1,2-dihydropyrimidin-2-one or salts thereof is in an amount from about 5 to about 1000 uM.

11. The use according to any of preceding claims, wherein 1-(β-D-Ribofuranosyl)-1,2-dihydropyrimidin-2-one or salts thereof is in an amount of from about 50 to about 200 uM.

12. Use according to any of preceeding claims, wherein said medicament is for the treatment of Rheumatoid arthritis, Diabetes mellitus type I, Sjögren's syndrome, Multiple Sclerosis, Crohn's disease, arteriosclerosis, Parkinson's disease, ALS (Amyotrophic lateral sclerosis) and dementia.

13. Use according to any of claims 1-11, for the manufacturing of a medicament to be used in transplantations to inhibit immune rejection of organs, tissues, normal or gene therapeutically modified cells.

## Patentansprüche

1. Verwendung von 1-(β-D-Ribofuranosyl)-1,2-dihydropyrimidin-2-on oder Salzen hiervon oder einem Analogon, wobei das genannte Analogon aus der Gruppe, bestehend aus 5-Methylcytidin, 2'-Desoxyzebularin, 5-Fluorzebularin, 5-Fluor-2'-desoxyzebularin, 5-Chlorzebularin, 5-Chlor-2'-desoxyzebularin- 5-Bromzebularin, 5-Brom-2'-desoxyzebularin, 5-Jodzebularin, 5-Jod-2'-desoxyzebularin, 5-Me-2'-desoxyzebularin oder Mono-, Di- oder Triphosphaten hiervon, ausgewählt ist, zur Herstellung eines Medikaments zur Behandlung einer Autoimmunstörung oder - erkrankung, ausgewählt aus der Gruppe, bestehend aus Achlorhydrie, akute hämorrhagische Leukenzyphalitis, Morbus Addison, Alopecia areata, Anämie, perniziöse Anti-Glomeruläre-Basalmembran-Erkrankung, Antiphospholipid-Syndrom, aplastische Anämie, atopische Allergie, autoimmune atrophische Gastritis, autoimmune Hörschädigung, autoimmunhämolytische Anämie, Autoimmunhypoparathyreoidismus, Autoimmunhypophysitis, Autoimmunlymphoproliferatives Syndrom, Autoimmunmyokarditis, autoimmune Oophoritis, Autoimmunorchitis, Autoimmun-Polyendokrinopathie-Candidiasis-Ektodermale Dystrophie, Autoimmunes Syndrom Typ II, polyglanduläres Syndrom, Morbus Behcet, Zöliakie, Morbus Chagas, Cholangitis, Sklerosierung, chronisch inflammatorische demyelinisierende Polyneuropathie, chronische lymphatische Thyreoiditis, Churg-Strauss-Syndrom, Kolitis, ulzerative Kolitis, Morbus Crohn, Kryoglobulinämie, Cushing-Syndrom, Morbus Duhring, Dermatomyositis, Diabetes mellitus (insulinabhängig), diffuse zerebrale Sklerose (Schilder), experimentelle autoimmune Enzephalomyelitis (EAE), Epidermolysis bullosa acquisita, Erythematodes, Felty-Syndrom, Glomerulonephritis (IGA), membranöse Glomerulonephritis, Goodpasture-Syndrom, Morbus Graves, Guillain-Barre-Syndrom, Hamman-Rich-Syndrom, autoimmune Hepatitis, chronisch aktive Hepatitis, idiopathische Thrombozytopenie, chronisch entzündliche Darmerkrankung, Insulinresistenz Typ B, Lambert-Eaton-Myasthenie-Syndrom, Linseneiweiß-induzierte Uveitis, Lichen sclerosus et atrophicus, discoide Lupus erythematodes, systemische Lupus erythematodes, Lupus Hepatitis, Lupus Nephritis, Lymphopenie, Morbus Meniere, kombinierte Erkrankung des Bindegewebes, Mooren-Ulkus, mukokutanes Lymphknotensyndrom, Multiple Sklerose, Myasthenie gravis, transverse Myelitis, Myokarditis, Narkolepsie, experimentelle autoimmune Neuritis, Neuromyelitis optica, Cogan-Syndrom, Ophthalmia sympathetica, Opsoclonus-Myoclonus-Syndrom, Pankreatitis, bullöses Pemphigoid, Pemphigus foliaceus, Pemphigus vulgaris, Polyarteriitis nodosa, rezidivierende Polychondritis, Autoimmun-Polyendokrinopathien, Polymyalgia rheumatica, Polyradikuloneuropathie, primäre billiäre Zirrhose, idiopathische thrombozytopenische Purpura, Raynaud-Syndrom, Morbus Reiter, rheumatisches Fieber, rheumatoide Arthritis, Sarkoidose, Sklerodermie, Sjögren-Syndrom, Spondylitis ankylosans, Stiff-Person-Syndrom, Morbus Still in der adulten Form, Takayasu-Arteriitis, Arteriitis temporalis, Thyreotoxikose, Typ B Insulinresistenz, Uveo-Meningoenzephalitis-Syndrom, Wegener-Granulomatose, Vitiligo oder Immunabstoßung an Transplantaten oder gentherapeutisch modifizierten Zellen, wobei die Behandlung die Indolamin-Dioxygenase induziert.

2. Verwendung gemäß Anspruch 1, wobei das genannte Medikament einen pharmazeutisch annehmbaren Puffer, Hilfsstoff, Verdünnungsmittel oder Träger enthält.

3. Verwendung gemäß einem der vorhergehenden Ansprüche, wobei das genannte Medikament wenigstens zwei oder mehr Vertreter von 1-(β-D-Ribofuranosyl)-1,2-dihydropyrimidin-2-on oder Salzen hiervon enthält.

4. Verwendung gemäß einem der vorhergehenden Ansprüche, wobei das genannte Medikament ein oder mehrere zusätzliche Wirkstoffe enthält.

5. Verwendung gemäß einem der vorhergehenden Ansprüche, wobei das genannte Medikament ferner wenigstens eine Verbindung, ausgewählt aus der Gruppe, bestehend aus Metaboliten von Tryptophan, Immunosuppressiva, HDAC-Inhibitoren und Substanzen zur Erhöhung der Bioverfügbarkeit und/oder zur Verringerung der Degradation von 1-(β-D-Ribofuranosyl)-1,2-dihydropyrimidin-2-on, enthält.

6. Verwendung gemäß Anspruch 5, wobei die genannte Verbindung aus der Gruppe, bestehend aus Aldehyd-Oxidase-Inhibitoren, ausgewählt aus der Gruppe, bestehend aus Raloxifen, Perphenazin, Thioridazin, Menadion, Trifluperazin, Amitriptylin, Estradiol, Felodipin, Chlomipramin, Loratidin, Promethazin, Chlorpromazin, Ethinylestradiol, Norclomipramin, Amodiaquin, Nortriptylin, um die Oxidation von Zebularin zu Uridin zu hemmen, ausgewählt ist.

7. Verwendung gemäß Anspruch 5, wobei die genannte Verbindung aus der Gruppe, bestehend aus Tryptophan, N-Formylkynurenin, Formylanthranilat, Anthranilat, L-Kynurenin, 4-(2-Aminophenyl)-2,4-dioxybutanoat, Kynurensäure, 3-Hydroxy-L-kynurenin, 3-Hydroxyanthranilat, 3-Methoxyanthranilat, 4-(2-Amino-3-hydroxyphenyl)-2,4-dioxobutanoat, Xanthurenat, 8-Methoxykynurenat, 2-Amino-3-carboxymuconat-semialdehyd, 2-Aminomuconat-semialdehyd, Chinolinsäure, Cinnavalininat, Tryptamin, N-Methyltryptamin, Indolacetat, 2-Formaminobenzoylacetat, 5-Hydroxy-L-tryptophan, 5-Hydroxy-N-formylkunerin, 5-Hydroxykunerin, 5-Hydroxykunerenamin, 4,6-Dihydroxychinolin, Serotonin, N-Acetylserotonin, Melatonin, 6-Hydroxymelatonin, Formyl-N-acetyl-5-methoxykynurenamin, N-Methylserotonin, Formyl-5-hydroxykynurenamin, 5-Methoxytryptamin, 5-Hydroxyindolacetaldehyd, 5-Hydroxyindolacetat, 5-Methoxyindolacetat oder 5-Hydroxyindolacetylglycin, ausgewählt ist.

8. Verwendung gemäß Anspruch 5, wobei die genannte Verbindung aus der Gruppe, bestehend aus Glykokortikoiden, Methotrexat, Rapamycin, Cyclophosphamid, Antimetaboliten, einschließlich Azathioprin, Immunophilin-bindende Wirkstoffe (einschließlich Toleranz, Tacrolismus, Sirolismus, Everolismus), Nukleotidsynthese-Inhibitoren (einschließlich Mycophenolat-Mofetil, Mizoribin, Leflunomid, FK778), FTY720, Lymphozyten-abbauende Antikörper (einschließlich polyklonale Antikörper gegen Lymphozyten, Thymozyten, T-Zellen, Muromonab-CD3, Rituximab, Alemtuzumab, CAMPATH-1), nicht-abbauende Antikörper (einschließlich Daclizumab, Basiliximab, die beiden CTLA4-Ig-Fusionsproteine LEA29Y und Abatacept, LFA3-Ig-Fusionsprotein), Anti-TNF-Antikörper (einschließlich Infliximab, Adalimumab), Natalizumab (Anti-VLA-4), die Anti-CD154-Antikörper BG9588 und IDEC 131), lösliche Zytokin-Rezeptoren (einschließlich Lenercept und Etanercept (lösliche TNF p55- und TNF p75-Rezeptoren), Histon-Deacetylase-Inhibitoren und Anakinra (lösliche IL-1RA), ausgewählt ist.

9. Verwendung gemäß einem der vorhergehenden Ansprüche, wobei das genannte Medikament eine Tablette, eine Kapsel, eine Lösung oder ein Pulver ist.

10. Verwendung gemäß einem der vorhergehenden Ansprüche, wobei 1-(β-D-Ribofuranosyl)-1,2-dihydropyrimidin-2-on oder Salze hiervon in einer Menge von etwa 5 bis etwa 1.000 µM vorliegt.

11. Verwendung gemäß einem der vorhergehenden Ansprüche, wobei 1-(β-D-Ribofuranosyl)-1,2-dihydropyrimidin-2-on oder Salze hiervon in einer Menge von etwa 50 bis etwa 200 µM vorliegt.

12. Verwendung gemäß einem der vorhergehenden Ansprüche, wobei das genannte Medikament für die Behandlung von rheumatoider Arthritis, Diabetes mellitus Typ I, Sjögren-Syndrom, Multiple Sklerose, Morbus Crohn, Arteriosklerose, Morbus Parkinson, ALS (amyotrophe Lateralsklerose) und Demenz, vorgesehen ist.

13. Verwendung gemäß einem der Ansprüche 1 bis 11 zur Herstellung eines Medikaments, das bei Transplantationen eingesetzt werden soll, um die Immunabstoßung von Organen, Geweben, normalen Zellen oder gentherapeutisch modifizierten Zellen zu hemmen.

## Revendications

1. Utilisation de 1-(β-D-ribofuranosyl)-1,2-dihydropyrimidin-2-one ou de sels de celle-ci ou d'un analogue, dans laquelle ledit analogue est choisi dans le groupe constitué par la 5-méthylcytidine, la 2'-désoxyzébularine, la 5-fluorozébularine, la 5-fluoro-2'-déxyzébularine, la 5-chlorozébularine, la 5-chloro-2'-déxyzébularine, la 5-bromozébularine, la 5-bromo-2'-déxyzébularine, la 5-iodozébularine, la 5-iodo-2'-déxyzébularine, la 5-Me-2'-désoxyzébularine, ou leurs mono-, di- ou tri-phosphates, pour la fabrication d'un médicament destiné au traitement d'une maladie ou d'un trouble auto-immun choisi dans le groupe constitué par l'achlorhydrie, la leuco-encéphalite hémorragique aiguë, la maladie d'Addison, la pelade, l'anémie pernicieuse, la glomérulonéphrite auto-immune, le syndrome des antiphospholipides, l'anémie aplasique, l'atopie, la gastrite atrophique auto-immune, la perte auditive auto-immune, l'anémie hémolytique auto-immune, l'hypoparathyroïdie auto-immune, l'hypophysite auto-immune, le syndrome lymphoprolifératif avec auto-immunité, la myocardite auto-immune, l'oophorite auto-immune, l'orchite auto-immune, la polyendocrinopathie-candidose-dystrophie ectodermique auto-immune, le syndrome de type II auto-immun, le syndrome de Behcet polyglandulaire, la maladie coeliaque, la maladie de Chagas, l'angiocholite sclérosante, la polyneuropathie démyélinante inflammatoire chronique, la thyroïdite lymphocytaire chronique, le syndrome de Churg-Strauss, la recto-colite hémorragique, la maladie de Crohn, la cryoglobulinémie, le syndrome de Cushing, la dermatite herpétiforme, la dermatomyosite, le diabète sucré (insulinodépendant), la sclérose cérébrale diffuse de Schilder, l'encéphalomyélite, l'épidermolyse auto-immune expérimentale (EAE), l'épidermolyse bulleuse acquise, le syndrome de Felty érythémateux, la glomérulonéphrite (IGA), la glomérulonéphrite membraneuse, le syndrome de Goodpasture, la maladie de Grave, le syndrome de Guillain-Barré, le syndrome de Hamman-Rich, l'hépatite auto-immune, l'hépatite chronique active, la thrombocytopénie idiopathique, les maladies intestinales inflammatoires, la résistance à l'insuline, le syndrome myasthénique de Lambert-Eaton de type B, l'uvéite due au port de lentilles, le lichen atrophique et scléreux, le lupus érythémateux discoïde, le lupus érythémateux disséminé, le lupus hépatique, le lupus néphritique, la lymphopénie, la maladie de Ménière, la connectivite mixte, l'ulcère de Mooren, le syndrome adénocutanéomuqueux, la sclérose en plaques, la myasthénie grave, la myélite transverse, la myocardite, la narcolepsie, la névrite auto-immune expérimentale, la neuromyélite optique, le syndrome vestibulo-oculaire et auditif, l'ophtalmie sympathique, le syndrome d'opsoclonie-myoclonie, la pancréatite, la pemphigoïde bulleuse, le pemphigus foliacé, le pemphigus vulgaire, la polyartérite noueuse, la polychondrite chronique, les polyendocrinopathies auto-immunes, la polymyalgie rhumatismale, la polyradiculonévrite, la cirrhose biliaire primitive, le purpura thrombocytopénique idiopathique, la maladie de Raynaud, le syndrome de Reiter, le rhumatisme articulaire aigu, la polyarthrite rhumatoïde, la sarcoïdose, la sclérodermie, le syndrome de Sjögren, la spondylarthrite ankylosante, le syndrome de Stiff-Person, la maladie de Still de l'adulte, l'artérite de Takayasu, l'artérite temporale, la thyrotoxicose, la résistance à l'insuline de type B, le syndrome d'uvéoméningite encéphalique, la granulomatose de Wegener, le vitiligo, ou le rejet immunitaire de greffes ou de cellules modifiées par traitement génique, et dans laquelle le traitement induit de l'indolamine dioxygénase.

2. Utilisation selon la revendication 1, dans laquelle ledit médicament comprend un tampon, excipient, diluant ou véhicule pharmaceutiquement acceptable.

3. Utilisation selon l'une quelconque des revendications précédentes, dans laquelle ledit médicament comprend au moins deux ou plus parmi la 1-(β-D-ribofuranosyl)-1,2-dihydropyrimidin-2-one et ses sels.

4. Utilisation selon l'une quelconque des revendications précédentes, dans laquelle ledit médicament comprend un ou plusieurs ingrédients actifs additionnels.

5. Utilisation selon l'une quelconque des revendications précédentes, dans laquelle ledit médicament comprend en outre au moins un composé choisi dans le groupe constitué par les métabolites du tryptophane, les agents immunodépresseurs, les inhibiteurs de HDAC et les substances servant à augmenter la biodisponibilité et/ou à réduire la dégradation de 1-(β-D-ribofuranosyl)-1,2-dihydropyrimidin-2-one.

6. Utilisation selon la revendication 5, dans laquelle ledit composé est choisi dans le groupe constitué par les inhibiteurs d'aldéhyde oxydase choisis dans le groupe constitué par le raloxifène, la perphénazine, la thioridazine, la ménadione, la triflupérazine, l'amitriptyline, l'oestradiol, la félodipine, la clomipramine, la loratidine, la prométhazine, la chlorpromazine, l'éthinyl-oestradiol, la norclomipramine, l'amodiaquine, la nortriptyline, pour inhiber l'oxydation de zébularine en uridine.

7. Utilisation selon la revendication 5, dans laquelle ledit composé est choisi dans le groupe constitué par le tryptophane, la N-formylkunurénine, le formylanthranilate, l'anthranilate, la L-kynurénine, le 2,4-dioxybutanoate de 4-(2-aminophényle), l'acide kynurénique, la 3-hydroxy-L-kynurénine, le 3-hydroxy-anthranilate, le 3-méthoxyanthranilate, le 2,4-dioxobutanoate de 4-(2-amino-3-hydroxyphényle), le xanthurénate, le 8-méthoxykurénate, l'hémialdéhyde de 2-amino-3-carboxymuconate, l'hémialdéhyde de 2-amino-muconate, l'acide quimolinique, le cinnavalininate, la tryptamine, la N-méthyltryptamine, l'acétate d'indole, l'acétate de 2-formaminobenzoyle, le 5-hydroxy-L-tryptophane, la 5-hydroxy-N-formylkunérine, la 5-hydroxykunérine, la 5-hydroxykunérénamine, la 4,6-dihydroxyquinoline, la sérotonine, la N-acétylsérotonine, la mélatonine, la 6-hydroxymélatonine, la formyl-N-acétyl-5-méthoxykynurénamine, la N-méthylsérotonine, la formyl-5-hydroxykynurénamine, la 5-méthoxytryptamine, le 5-hydroxyindole-acétaldéhyde, l'acétate de 5-hydroxyindole, l'acétate de 5-méthoxyindole, et la 5-hydroxyindole-acétylglycine.

8. Utilisation selon la revendication 5, dans laquelle ledit composé est choisi dans le groupe constitué par les glucocorticoïdes, le méthotrexate, la rapamycine, le cyclophosphamide, les antimétabolites, y compris l'azathioprine, les médicaments se liant à l'immunophiline (y compris la tolérance, le tacrolimus, le sirolimus, l'évérolimus), les inhibiteurs de la synthèse de nucléotides (y compris le mycophénolate mofétil, la mizoribine, le léflunomide, FK778), FTY720, les anticorps diminuant les lymphocytes (y compris les anticorps polyclonaux dirigés contre les lymphocytes, les thymocytes, les cellules T, le muromonab-CD3, le rituximab, l'alemtuzumab, CAMPATH-1), les anticorps non réducteurs (y compris le daclizumab, le basiliximab, les deux protéines de fusion de CTLA-4-Ig LEA29Y et abatacept, la protéine de fusion de LFA3-Ig), les anticorps anti-TNF (y compris l'infliximab, l'adalimumab), le natalizumab (anti-VLA-4), les anticorps anti-CD154 BG9588 et IDEC 131), les récepteurs de cytokines solubles (y compris le lénercept et l'étanercept (récepteurs de TNF p55 et de TNF p75 solubles)), les inhibiteurs d'histone désacétylase, et l'anakinra (IL-1RA soluble).

9. Utilisation selon l'une quelconque des revendications précédentes, dans laquelle ledit médicament est un comprimé, une capsule, une solution ou une poudre.

10. Utilisation selon l'une quelconque des revendications précédentes, dans laquelle la 1-(β-D-ribofuranosyl)-1,2-dihydropyrimidin-2-one ou ses sels est présente en une quantité d'environ 5 à environ 1000 µM.

11. Utilisation selon l'une quelconque des revendications précédentes, dans laquelle la 1-(β-D-ribofuranosyl)-1,2-dihydropyrimidin-2-one ou ses sels est présente en une quantité d'environ 50 à environ 200 µM.

12. Utilisation selon l'une quelconque des revendications précédentes, dans laquelle ledit médicament est destiné au traitement de la polyarthrite rhumatoïde, du diabète sucré de type I, du syndrome de Sjögren, de la sclérose en plaques, de la maladie de Crohn, de l'artériosclérose, de la maladie de Parkinson, de la SLA (sclérose latérale amyotrophique), et de la démence.

13. Utilisation selon l'une quelconque des revendications 1 à 11, pour la fabrication d'un médicament destiné à être utilisé lors de transplantations pour inhiber le rejet immunitaire d'organes, de tissus, ou de cellules normales ou modifiées par traitement génique.
